# EUROPEAN PATENT APPLICATION

(11) **EP 1 264 839 A1**
(43) Date of publication of application: **11.12.2002**
(21) Application number: 01113909.4
(22) Date of filing: 07.06.2001
(51) Int. Cl.: C07K 1/26, C07K 1/28, C07K 1/36

(54) **Method for separating protein mixtures**

(71) Applicant: ProteoSys AG, 55116 Mainz (DE)
(72) Inventor: Wozny, Wojciech, 55268 Nieder Olm (DE); Cahill, Michael A., 72116 Mössingen (DE)
(74) Representative: Patentanwälte Ruff, Wilhelm, Beier, Dauster & Partner

(57) **Abstract**

A method for separating protein mixtures is provided. The inventive method is especially useful in characterising and/or identifying low abundance proteins of interest form complex mixtures of protein, such as from human whole cell lysate protein mixtures, in the order of one to several attomole of target protein. After labelling proteins of a protein mixture the mixture is separated and subsequently at least one population of labelled proteins (e.g. a protein spot from two dimensional gel electrophoresis) is isolated from the protein mixture (e.g. by two dimensional gel electrophoresis). This isolated protein population is mixed with a protein mixture containing an excess amount of the same protein in unlabelled form. After further biochemical fractionation of the resulting protein mixture, fractions containing the labelled target protein of interest are identified by tracking the label, and unlabelled protein identical to the labelled protein is characterised and/or identified. The success of method is dependent upon comigration of identical labelled and unlabelled proteins during the biochemical fractionation procedure employed. Preferably the labelling of the proteins is performed by radioactive labelling, especially by radioiodination. The method is applicable to any analyte molecules, not just proteins.

## Description

The present invention relates to a method for separating protein mixtures.

In the field of protein biochemistry, the investigation of all proteins expressed by the genome of the cells under study has recently been of considerable interest. This branche of biochemistry is called proteomics, wherein this means PROTEins expressed by a genOME. Current proteomics techniques involve the use of two-dimensional polyacrylamide gel electrophoresis (2D-PAGE), whereby proteins are initially separated by differences in their isoelectric point (pl) by isoelectric focussing (IEF) in the first dimension. These proteins are then subsequently separated by molecular weight in an SDS-PAGE in the second dimension. Other methods, such as capillary electrophoresis, micro-separation devices, direct characterisation of proteins by mass spectrometry without prior gel separation, or using arrays of affinity reagents, are under investigation. Yet to date 2D-PAGE provides the most efficient separation of complex mixtures of proteins.

The aim of proteomics studies is to analyse and characterise all proteins of the cell. A further aim is to compare proteomics data to other measured cellular parameters in order to understand, model, and predict cellular or organismal responses. This means that both proteins which are very abundant in the cell, as well as proteins of very low abundance, have to be investigated. Especially the investigation of very low abundant proteins leads to several problems in proteomics studies. The crucial point is that low abundant proteins are getting lost during handling of the sample, for example due to non specific sticking of the low abundant proteins to the walls of reaction vessels or pipette tips, for example. Therefore, up to now it is only possible to detect and identifiy, and characterise the state of post-translational modification of proteins in the order of about 0.1 to 1 picomol protein.

This limit is a severe problem because many low abundant proteins are of special interest in protein biochemistry and proteomics studies. The most interesting proteins of a cells are often signal transduction molecules and other regulating proteins, which are present in very low copy numbers. These low abundant proteins are very interesting candidates when investigating changes in protein level in pathological situations. Therefore, several attempts were made to make these proteins available for analysis. One approach is to start the separation procedures with a large amount of protein mixture. Nevertheless, in general this leads to an extreme overload of separation means, for example an overload of polyacrylamid gels, leading to an overheating during electrophoresis and very poor resolution. Furthermore the low abundant proteins are covered and masked by higher abundant proteins and are not visible and thus not available for analysis. The present invention relies on the existence of a method to separate at least some low abundance to homogeneity or near homogeneity. Very high resolution 2D-PAGE is suitable.

Therefore the invention has the object to provide a method for separating protein mixtures, which allows the characterisation and identification of low abundant proteins. By use of the inventive method it should be possible to analyse proteins in amounts as low as sub-femtomole and even attomole concentrations. This objective is solved by a method as described in claim 1. Prefered embodiments of the inventive method are claimed in the dependent claims 2 to 16. The wording of all claims is hereby made to the content of the specification by reference.

The inventive method for separating protein mixtures is characterized by at least the following steps:
a) labelling of proteins (at least one) of a protein mixture,
b) separating said protein mixture,
c) isolating at least one labelled purified or partially purified protein from the protein mixture,
d) mixing the isolated protein with a (considerably) higher, preferably a multiple amount of the same protein in unlabelled form or with a protein mixture, comprising said higher or multiple amount of the same protein in unlabelled form,
e) separating said protein mixture resulting from step d) and
f) characterizing and/or identifying the unlabelled protein molecules which co-fractionate with the labelled protein, and optionally
g) ideally confirming identity of identified proteins by independent means, such as immunologically.

Briefly, a labelled low abundant protein is isolated and mixed with a (large) excess of biochemically identical or similar protein, which is not labelled. Then this mixture is biochemically fractionated to retrieve the fraction containing the protein of interest. Due to the vast excess of biochemically similar and unlabelled protein, the non specific loss of the labelled protein, for example by surface absorption etc., is negligible. This inventive method allows the analysis of low abundant proteins in amounts of one attomol or less. This method facilitates the detection of proteins at extremely low copy numbers per cell, commencing from low microgram amounts of labelled protein, for example about 15 µg.

In general, all common labelling methods are suitable for the inventive method, for example fluorescence labelling or luminescence labelling. Especially preferred is the application of radioactive labelling, because this is at the moment the most sensitive way to label a protein. It is possible to label the protein mixture by metabolically labelling methods or by in vitro labelling methods known to an expert in the art. Especially preferred the proteins are radioiodinated. Because of the long half-life period of radioactive iodine (60 days) the labelled protein is stable over a long period. Preferably the iodination is performed by common methods like the iodogen method and unincorporated iodine is removed by gel filtration. In a preferred embodiment less than 100 µg are used as starting material for the labelling, especially preferred is the use of less than about 10 µg. Labelling of proteins with iodine leads to a labelling of almost all proteins of the protein mixture. Proteins under 30 kDa have more chance of containing no cysteine or tyrosine residues. On average proteins are typically labelled once per 40 kDa, or approximately once per 360 amino acids, however this does not limit the method. A slight difference this has on the overall biochemical properties of the proteins is insignificant, as they cofractionate with non-labelled proteins in biochemical fractionation. According to the invention it is possible to label as little as 1 µg of total protein with no qualitative loss of separating pattern complexity.

In a preferred embodiment of the invention the first separating of the protein mixture comprising the labelled proteins according to step b) is performed in at least two dimensions, wherein preferably one dimension is an isoelectric focussing (IEF) and/or one dimension is a sodiumdodecylsulfate polyacrylamide gel electrophoresis (SDS-PAGE). The serial combination of IEF and SDS-PAGE forms the method of two-dimensional gel electrophoresis (2D-PAGE), which is commonly used in the field of proteomics. Preferably, the first dimension IEF is especially long in order to obtain a good resolution of the separation. After running the IEF gel it is preferably cut into smaller strips for the second SDS-PAGE dimension, or can be electrophoresed in an extremely long SDS-PAGE gel. According to the invention any separation method can be used for this separating step as long as the protein mixture is sufficiently separated into distinct proteins which can be isolated.

The isolation of at least one labelled protein from the protein mixture according to step c) is preferably performed by physically removing the labelled protein as a protein spot from a polyacrylamide gel, especially from a 2D-PAGE. In a preferred embodiment the spot is simply cut out from the gel for example by the use of a scalpel or automated device. Before removing the labelled protein from the gel the corresponding protein is detected dependent from the chosen labelling method. For example the labelled proteins are detected by phosporimaging or by radioimaging by a multiple photon detection (MPD) imager, or a chamber containing ionisable gas. In a preferred embodiment a low abundance protein is taken from a two-dimensional gel. It is possible, that the corresponding protein spot contains one picomol or less, even one attomol of label, for example ¹²⁵I.

The isolated labelled protein is then mixed with another (unlabelled) biochemically identical or similar protein or a protein mixture, at least comprising a biochemically idendical or similar protein. The unlabelled protein prevents the non specific loss of the labelled protein, because the unlabelled protein is added in excess, preferably in vast excess. According to the invention the protein mixture, which is added according to step d), has to comprise the same protein as the labelled protein, or the biochemically similar protein is added alone, if available. Preferably a large excess of protein from a source known to contain protein of interest is added. In an especially preferred embodiment a large excess of protein from the same source as the labelled protein is added, because in this embodiment it is not necessary to know further details about the nature of the labelled protein. As an example about 500 mg or more of protein from the same source is added to the isolated labelled protein spot containing ¹²⁵I in the order of attomol or picomol. It is also possible that gel pieces containing attomol or picomol quantities of labelled proteins from more than one gel are excised, and mixed with about 500 or more mg of protein.

According to the inventive method the protein mixture resulting from the mixing step is furthermore separated in at least one and preferably in at least two separation steps using prior art biochemical fractionation techniques, such as chromatography, gel filtration, free flow electrophoresis, or other techniques known to an expert in the art. After or during one or more, preferably each separation step the protein mixture is fractionated and the different fractions are analysed with respect to the presence of the labelled protein of interest. The fraction or fractions containing the labelled protein are used for further separation steps. The detection of the labelled protein during the separation process or within the different fractions is performed by methods known to an expert in the art dependent from the label used for the labelling of proteins according to step a). In the preferred case when the proteins are labelled radioactively the protein of interest is detected by common scintillation counter, for example a crystal scintillation gamma counter. In an especially preferred embodiment of the invention the protein of interest is detected by multiple photon detection (MPD). This technology permits the detection and measurement of certain radioisotopes which decay by the electron capture mechanism and emit multiple photons, at levels that are much below the naturally occuring background radiation. Background activity rarely provides coincident photons of defined energies, and can be excluded from the analysis. The nearly perfect rejection of background events is achieved through sophisticated signal processing and analysis. The high degree of sensitivity of MPD is obtained by considering only signals with coincident X-and Y-rays and where the energy profiles of the measured photons correspond to those expected for the isotope in question. The isotopes compatible with MPD include the isotope ¹²⁵I, one of the most commonly used isotopes in biomedical diagnostics and especially in the present inventive method. Additionally, other iodine isotopes can also be detected by MPD analysis. In comparison with common radioactive scintillation counting MPD is approximately 100 times more sensitive.

An MPD detector consists of two detectors, one of each side of the source, with each able to detect independent photons. Four isotopes of iodine (¹²⁴I, ¹²⁵I, ¹²⁶I and ¹³¹I) are compatible with MPD for protein labelling.

MPD detectors include sequential sampling MPD (ssMPD) instruments, which function analogously to conventional Cerenkov counters. ssMPD functions analogously to a conventional γ-counter, but is 100 times more sensitive and can detect zeptomol quantities of ¹²⁵I. Therefore, the supersensitive MPD counters, and especially the ssMPD counters, are preferred as detection means in the inventive method. By one such counter it is possible to select fractions containing more than 0.1 attomol ¹²⁵I from 96 fractions in about 24 hours. By counting the fractions by ssMPD it is possible to obtain proteins originally present at one attomol or even less in quantities of one picomole or more. By the inventive method it is possible to identify and characterise with certainty proteins present at 100 copies per cell, for which it is needed about one picomole of protein. For this about 500 mg of whole mammalian cell extract proteins are necessary to provide one picomol of 100-copy per cell proteins. Because it is possible by the inventive method to start with 10 femtomol of protein it is possible to identify protein species above the 1-copy per mammalian cell level with the inventive system. Such molecules with extremely low abundance are for example of interest, if trace amounts of molecules in body fluids are to be investigated.

In a preferred embodiment of the inventive method the separating of the protein mixture comprising the isolated labelled protein and the multiple amount of the same protein according to step e) is performed in at least one SDS-PAGE step. Advantageously this SDS-PAGE is a preparative SDS-PAGE, for example an SDS-PAGE column. One example of such a suited column is the bio-Rad model 491 Prep Cell device, which can be loaded, e. g., with 500 mg of cell extract in 50 ml of SDS/Tris buffer. By such a Laemmli SDS-PAGE procedure molecules are separated which differ in apparent molecular weight by as little as 2 %, and are obtained as liquid fractions at the end of the gel, which are preferably counted by ssMPD.

In a preferred embodiment of the inventive method the separating according to step e) of the inventive method is performed by at least one IEF, especially a preparative IEF, like a free flow IEF. In an especially preferred embodiment the fractions obtained by a preparative SDS-PAGE, containing the labelled protein, as described above, are loaded onto an appropriate IEF device following buffer and detergent exchange. Obviously, it is possible to use all common IEF devices, known to an expert in the art. Depending on the actual separating conditions it could be preferable to change the buffer conditions of the fractions containing the labelled protein after the first separation step before starting the second separation step.

Especially preferred is the combination of SDS-PAGE, especially preparative SDS-PAGE, and IEF, especially preparative IEF, such as free flow electrophoresis. According to the invention, the labelled proteins of interest, for example iodinated proteins, comigrate with the non-labelled proteins in 2D-PAGE. The small differences in molecular weight and pl caused by the labelling, especially the iodination, are not measurable.

There are two established methods for IEF used for 2D-PAGE. The first system developed employs an initial equilibrium focussing of carier ampholytes to their isoelectric point, followed by separation of proteins in a second dimension according to their migration in SDS-PAGE. The resolving power of this system is excellent, with IEF gels up to about 45 cm reported and over 10,000 protein spots per 2D-PAGE gel. However, the basic region of the pH gradient migrates out of the gel before equilibrium is reached during electrophoresis, because of cathodic instability caused by buffering from the bicarbonate ion, which is in equilibrium with atmospheric carbon dioxide. In order to analyse proteins in the basic pH region, present carrier ampholyte IEF experiments are stopped before equilibrium, while the pH gradient is not yet complete, but while the basic region is still in the gel. This non-equilibrium pH gradient gel electrophoresis (NEPHGE) is almost the only way to analyse basic proteins with the carrier ampholyte-based gel electrophoresis system.

The second IEF system utilises acrylamide-like acrylamido buffer monomers (immobilines) which contain a functional buffering group. A concentration gradient of buffer molecules is formed, followed by polymerisation of the polyacrylamide matrix, whereby the buffering molecules are co-polymerized into the matrix, and thus immobilized. These immobilized pH gradient (IPG) gels are commonly cast using density gradient of glycerol between the two solutions of different pH.

Since this is diffusion limited, immobiline gradients in the liquid phace are typically less than 30 cm in length such that the density gradient can form.

In an especially preferred embodiment for the performance of IEF the acrylamide-like monomer N-acryloylaminopropanol (AAP) or Duracryl are used for "polyacrylamide-gels". These monomers form a more hydrophilic matrix than polyacrylamide. In a further preferred embodiment the "polyacrylamide" matrix is co-polymerized with polyethylene glycol to increase the pore size of the gel matrix.

In another preferred embodiment of the inventive method the protein mixtures, especially fractions obtained by a first separation step, are electrophorized on narrow pH range IPG "zoom gels". By this electrophoresis procedure the resolution of the separation step is further improved.

In another embodiment of the inventive method the resolution of the electrophoresis is improved by extended distances for IEF or SDS-PAGE or in general for all polyacrylamide gels. Namely polyacrylamide gels of large dimensions for the separation of step b) and/or step e) are used, wherein preferably the length of the gel is at least about 50 cm, especially at least about 100 cm. In an especially preferred embodiment the length of the gel is about 500 cm. This permits a majority of protein species expected to be found in a mammalian cell tissue to be successfully resolved to homogeneity. 500 mm IEF gels deliver suitable separation performance to extract the maximum benefit from the detection sensitivity of radioactive methods.

In order to prepare appropriate polyacrylamide gels immobiline gradients are cast in an appropriate chamber, for example. After polymerisation, free acrylamide monomers are electrophoretically moved by addition of low concentration of cationic scavenger molecules, such as thioglycolate, to the anode chamber. In an especially preferred embodiment methylene blue-catalysed acrylamide polymerisation for capillary cast IPG gels is used, following casting of the gradient.

In the last step of the inventive method the labelled proteins are characterized and/or identified. In a preferred embodiment of the inventive method this last step is performed by at least one 2D-PAGE step. For example, the liquid fraction containing the labelled protein, obtained by a preparative IEF of step e) is loaded onto a IEF polyacrylamide gel. After electrophoresis the IEF gel is cut and electrophoresed in a SDS-PAGE dimension according to standard protocols. By this procedure the exact size and isoelectric point of the protein on question is estimated and thus the protein could be identified.

In another preferred embodiment of the inventive method the characterisation and/or identification is performed by mass spectrometry methods. Preferably the proteins are suggested by common methods known to an expert in the art and analysed by common methods. In an especially preferred embodiment the identification of the comigrating non-labelled protein is performed by aminoacid sequencing by chemical or mass spectrometric means.

In one especially preferred embodiment the characterization and/or identification of the labelled protein is performed by analysing posttranslational modifications of the protein. In general many proteins are modificated after their synthesis. For example, certain side chains of the proteins are changed, for example by introduction of carbohydrate units, a so-called glycosylation. Preferably these modifications are analysed by the inventive method in the last step. Knowledge about the specific modifications of a protein gives an important hint to the function and further characterisation of the protein of interest. Because the investigation of posttranslational modification requires under typical circumstances larger protein amounts, it could be preferred to perform the inventive method with more protein than described above, especially a larger amount of protein, which is added in step d) of the inventive method.

In one especially desired embodiment of the invention the cellular interaction partner molecules of the labelled target protein molecules are identified and characterised. It is generally recognised that protein interactions control protein localisation, and that the activity of proteins within cells is largely dependent upon their localisation, and hence interactions with other proteins, or with nucleic acids or other macromolecules.

The invention can be summarized as follows. A method for separating protein mixtures is provided. The inventive method is especially useful in characterising and/or identifying low abundance proteins of interest form complex mixtures of protein, such as from human whole cell lysate protein mixtures, in the order of one to several attomole of target protein. After labelling proteins of a protein mixture the mixture is separated and subsequently at least one population of labelled proteins (e.g. a protein spot from two dimensional gel electrophoresis) is isolated from the protein mixture (e.g. by two dimensional gel electrophoresis). This isolated protein population is mixed with a protein mixture containing an excess amount of the same protein in unlabelled form. After further biochemical fractionation of the resulting protein mixture, fractions containing the labelled target protein of interest are identified by tracking the label, and unlabelled protein identical to the labelled protein is characterised and/or identified. The success of method is dependent upon comigration of identical labelled and unlabelled proteins during the biochemical fractionation procedure employed. Preferably the labelling of the proteins is performed by radioactive labelling, especially by radioiodination. The method is applicable to any analyte molecules, not just proteins.

## Claims

1. Method for separating protein mixtures, at least comprising the following steps:
a) labelling of at least one target protein of a protein mixture,
b) separating the protein mixture,
c) isolating at least one labelled target protein from the protein mixture,
d) mixing the isolated target protein with a higher, preferably a multiple amount of the same protein in unlabelled form or with a protein mixture, comprising said higher or a multiple amount of the same protein in unlabelled form,
e) separating the protein mixture provided in step d) and
f) characterizing and/or identifying the unlabelled target protein which is identical to and biochemically comigrates with the labelled target protein during biochemical separation steps employed
g) Optionally confirming the identity of the target protein by any means available.

2. Method according to claim 1, **characterized in that** the labelling of step a) is a radioactive labelling, especially a radioiodination.

3. Method according to claim 1 or claim 2, **characterized in that** the separating of step b) is performed in at least two dimensions, wherein preferably one dimension is an isoelectric focussing (IEF) and/or one dimension is a SDS polyacrylamide gel electrophoresis (SDS-PAGE).

4. Method according to one of the preceding claims, **characterized in that** the isolating of step c) is performed by physically removing, especially cutting, the labelled protein from a polyacrylamide gel, especially a two-dimensional polyacrylamide gel electrophoresis (2D-PAGE).

5. Method according to one of the preceding claims, **characterized in that** for the mixing of step d) a protein mixture of the same source as the protein mixture of step a) is used.

6. Method according to one of the preceding claims, **characterized in that** the separating of step e) is performed in at least one and preferably in at least two separation steps.

7. Method according to claim 6, **characterized in that** after or during one or more separation steps the protein mixture is fractionated and the fraction or fractions containing the labelled protein are detected and used for at least one further separation step or for characterisation or identification of the target protein.

8. Method according to one of the preceding claims, **characterized in that** the labelled protein is detected by multiple photon detection (MPD), especially by sequential sampling multiple photon detection (ssMPD).

9. Method according to one of the preceding claims, **characterized in that** the separating of step e) is performed by at least one SDS-PAGE, especially a preparative SDS-PAGE, preferably an SDS-PAGE column.

10. Method according to one of the preceding claims, **characterized in that** the separating of step e) is performed by at least one IEF, especially a preparative IEF, preferably a free flow IEF.

11. Method according to one of the preceding claims, **characterized in that** for the separating of step b) and/or step e) polyacrylamide gels of large dimensions are used, wherein preferably the length of the gel is at least about 50 cm, especially over at least 100 cm.

12. Method according to one of the preceding claims, **characterized in that** the separation step e) is performed by at least one electrophoresis step.

13. Method according to one of the preceding claims, **characterized in that** the separation step e) is performed by at least one chromatography or gel filtration step.

14. Method according to one of the preceding claims, **characterized in that** the characterizing and/or identifying of step f) is performed by mass spectrometry, immunology, or chemical degradation methods.

15. Method according to one of the preceding claims, **characterized in that** the characterizing and/or identifying of step f) is performed by analysing posttranslational modifications of proteins.

16. Method according to one of the preceding claims, **characterized in that** the characterizing and/or identifying of step f) is used to subsequently identify other macromolecules which physically interact with or associate with the target proteins to affect their biological funtions.
